(19) Europäisches Patentamt
European Patent Office
Office européen des brevets

(11) Publication number: 0 219 906
B1

(12) EUROPEAN PATENT SPECIFICATION

(45) Date of publication of patent specification: 06.06.90

(51) Int. Cl.[5]: C 07 C 67/38, C 07 C 69/28 // C07C51/54, C07C53/122

(21) Application number: 86201759.7

(22) Date of filing: 13.10.86

# (54) Process for the preparation of alkylidene diesters.

(30) Priority: 14.10.85 GB 8525301

(43) Date of publication of application:
29.04.87 Bulletin 87/18

(45) Publication of the grant of the patent:
06.06.90 Bulletin 90/23

(84) Designated Contracting States:
BE CH DE FR GB IT LI NL

(56) References cited:
EP-A-0 106 379
US-A-3 720 706

(73) Proprietor: SHELL INTERNATIONALE RESEARCH MAATSCHAPPIJ B.V.
Carel van Bylandtlaan 30
NL-2596 HR Den Haag (NL)

(72) Inventor: Drent, Eit
Badhuisweg 3
NL-1021 CM Amsterdam (NL)

(74) Representative: Aalbers, Onno et al
P.O. Box 302
NL-2501 CH The Hague (NL)

EP 0 219 906 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for the preparation of an alkylidene diester.

It is known that olefins may be carbonylated in the presence of water, alcohols or carboxylic acids to yield carboxylic acids, esters or carboxylic acid anhydrides, respectively. However, the known processes have various drawbacks which render them rather unattractive for use on a technical scale.

United States patent specification No. 3,720,706 proposes that certain alkylidene diesters may be prepared by reaction of a carboxylic acid, an olefin, carbon monoxide and an aldehyde in the presence of certain catalyst systems comprising a Group VIII transition metal compound and an iodide promoter. The examples only illustrate the use of a catalyst system consisting of $[Rh(CO)_2Cl]$ dimer and ethyl iodide.

It is known from European Patent Specification 106,379 that ethylenically unsaturated compounds can be reacted with carbon monoxide and a carboxylic acid with formation of a carboxylic anhydride. This known reaction is carried out in the presence of a catalytic system prepared by combining palladium or a palladium compound, at least 5 mol of a phosphine $PR^1R^2R^3$ in which $R^1$, $R^2$ and $R^3$ each represent an optionally substituted aryl group and an acid with a $pK_a<2$ (at 18°C in aqueous solution), except hydrohalogenic and carboxylic acids.

It has now, surprisingly, been found that the same catalytic system can be used to convert an aldehyde with very rapid and selective formation of an alkylidene diester. The selectivity to a certain compound, expressed in a percentage, is defined herein as $100\times a/b$ in which "a" is the amount of the starting compound that has been converted into that certain compound and "b" is the total amount of the starting compound that has been converted.

Accordingly, the invention provides a process for the preparation of an alkylidene diester, which process comprises reacting an ethylenically unsaturated compound with carbon monoxide, a carboxylic acid and an aldehyde in the presence of a catalytic system prepared by combining palladium or a palladium compound, at least 5 mol of a phosphine $PR^1R^2R^3$ in which $R^1$, $R^2$ and $R^3$ each represent an optionally substituted aryl group, per gram atom of palladium, and an acid with a $pK_a<2$ (at 18°C in aqueous solution) as a promoter, except hydrohalogenic and carboxylic acids.

The acids used as promoters in the process according to the invention preferably have a non-co-ordinating anion, by which is meant that little or no co-valent interaction takes place between the palladium and the anion. Typical examples of such anions are $PF_6^-$, $SbF_6^-$, $BF_4^-$ and $ClO_4^-$.

Acids preferably used are, for instance sulphonic acids and those acids that can be formed, possibly in situ, by interaction of a Lewis acid such as, for example, $BF_3$, $AsF_5$, $SbF_5$, $PF_5$, $TaF_5$ or $NbF_5$ with a Broensted acid such as, for example, a hydrohalogenic acid, in particular HF, fluorosulphonic acid, phosphoric acid or sulphuric acid. Specific examples of the last-named type of acids are fluorosilicic acid, $HBF_4$, $HPF_6$ and $HSbF_6$. Typical sulphonic acids that can be used are fluorosulphonic acid, chlorosulphonic acid and the sulphonic acids specified hereinafter.

A preferred group of acids has the general formula

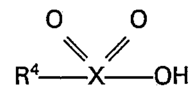

I

wherein X represents sulphur or chlorine and, if X is chlorine, $R^4$ represents oxygen and if X is sulphur, $R^4$ represents an OH group or an optionally substituted hydrocarbon group.

When the afore-mentioned acids are used in the process according to the invention, the anions of the acids can be regarded as non-co-ordinating.

The carbonylation of the ethylenically unsaturated compounds should be carried out in the presence both of an acid as hereinbefore defined and of at least 5 mol of the phosphine mentioned.

In the acids having the general formula I, the optionally substituted hydrocarbon group, represented by $R^4$, is preferably an alkyl, aryl, aralkyl or alkaryl group with 1—30, in particular 1—14, carbon atoms. The hydrocarbon group may be substituted with for instance halogen atoms, in particular fluorine atoms. Examples of suitable acids of the general formula I are perchloric acid, sulphuric acid, 2-hydroxypropane-2-sulphonic acid, p-toluenesulphonic acid and trifluoromethane sulphonic acid with the latter two acids being preferred. The acid of the general formula I may also be an ion exchange material which comprises sulphonic acid groups such as Amberlite 252 H. In this case the hydrocarbon group $R^4$ is a polymeric hydrocarbon group substituted with sulphonic acid groups, for instance a polystyrene group.

The quantity of the acid with a $pK_a<2$ present in the reaction mixture is preferably 0.01—150, more particularly 0.1—100, and most preferably 1—50 equivalents per gram atom of palladium. The acid can optionally be formed in situ, for example by hydrolysis of an ester, such as for instance an alkyl ester of a sulphonic acid, or by reacting a ketone with $SO_2$ and water.

The ethylenically unsaturated compound may be an unsubstituted or a substituted alkene or cycloalkene preferably having 2—30, and in particular 2—20, carbon atoms per molecule and preferably 1—3 double bonds per molecule. The alkene or cycloalkene may be substituted, for instance, with one or more halogen atoms or cyano, ester, alkoxy or aryl groups. Examples of suitable ethylenically unsaturated compounds are ethene, propene, 1-butene, 2-butene, isobutene, the isomeric pentenes, hexenes, octanes and dodecenes, 1,5-cyclooctadiene, cyclododecene, 1,5,9-cyclododecatriene, methyl acrylate, ethyl

acrylate, methyl methacrylate, acrylonitrile, vinyl chloride, allyl chloride, methyl allyl ether and styrene.

The carboxylic acids used in the process according to the invention may be aliphatic, cycloaliphatic or aromatic and may be substituted with one or more substituents, such as mentioned hereinbefore in connection with the ethylenically unsaturated compounds to be used as starting material. The carboxylic acids preferably contain not more than 20 carbon atoms per molecule. Examples of suitable carboxylic acids are acetic acid, propionic acid, butyric acid, caproic acid, trimethylacetic acid, benzoic acid, caprylic acid, succinic acid and adipic acid. Special preference is given to carboxylic acids having 1—10 carbon atoms per molecule. If the carboxylic acid has more than one carboxy group per molecule, different products may be formed, depending on the molar ratios existing between the reagents.

The aldehydes used in the process according to the invention may be aliphatic, cycloaliphatic or aromatic and may be substituted with one or more substituents, such as mentioned hereinbefore in connection with the ethylenically unsaturated compounds to be used as starting material. The aldehydes preferably contain 2—20 carbon atoms per molecule and are preferably alkanals. Examples of suitable aldehydes are ethanal, propanal, butanal, 2-methylbutanal, undecanal, benzaldehyde and anisaldehyde.

The quantity of aldehyde is not critical. Preference is given to the use of quantities in the range of from 0.5 to 5 mol aldehyde per mol carboxylic acid, but this molar ratio may very well be lower than 0.5 or higher than 5.

Reaction of an alkanecarboxylic acid having n+1 carbon atoms per molecule with carbon monoxide and an alkene having n carbon atoms per molecule yields in situ the symmetrical anhydride of the alkanecarboxylic acid having n+1 carbon atoms per molecule. This anhydride reacts in situ with an aldehyde with a surprisingly selective formation of an alkylidene diester. For example, propionic acid, ethylene and carbon monoxide form in situ propionic anhydride, which, in turn, reacts in situ with acetaldehyde with formation of ethylidene dipropionate, or, in turn, reacts in situ with propionaldehyde with formation of propylidene dipropionate. Ethylidene dipropionate may be used as a starting material for organic synthesis. For example, it may be decomposed by heating with formation of vinyl propionate (and propionic acid) from which polyvinyl esters can be prepared. The process thus leads to the conversion of an ethylenically unsaturated compound having n carbon atoms per molecule into a carboxylic anhydride having 2 n+2 carbon atoms per molecule, which, in situ, is converted by reaction with an aldehyde having m carbon atoms per molecule into an alkylidene diester of which the alkylidene moiety has m carbon atoms and each ester moiety has n+1 carbon atoms.

Both homogeneous and heterogeneous palladium compounds may be used in preparing the catalyst system according to the invention. However, homogeneous compounds are preferred. Suitable homogeneous compounds are the salts of palladium with, for instance, nitric acid, sulphuric acid or alkanecarboxylic acids having not more than 12 carbon atoms per molecule. Salts of hydrohalogenic acids may, in principle, be used as well, but they have the drawback that the halogen ion may have a corrosive effect. A compound used by preference is palladium acetate. Moreover, palladium complexes may be used, for instance palladium acetylacetonate, tetrakis-triphenylphosphinepalladium, bis-tri-o-tolylphosphinepalladium acetate or bis-triphenylphosphinepalladium sulphate. Palladium on charcoal and palladium bonded to an ion exchanger—for instance an ion exchanger comprising sulphonic acid groups—are examples of suitable heterogeneous compounds.

The quantity of palladium is not critical. Preference is given to the use of quantities in the range between $10^{-5}$ and $10^{-1}$ gram atom palladium per mol of ethylenically unsaturated compound.

The substituted or unsubstituted aryl groups $R^1$, $R^2$ and $R^3$ of the phosphine $PR^1R^2R^3$ preferably contain not more than 18, in particular 6—14, carbon atoms. Examples of suitable $R^1$, $R^2$ and $R^3$ groups are the naphthyl group and in particular the phenyl group. It has, surprisingly, been found that the process according to the present invention proceeds with a considerably higher reaction rate when each of the aryl groups carries an electron-withdrawing substituent. Examples of electron-withdrawing substituents are halogen atoms (fluorine, chlorine, bromine and iodine atoms), trihalogenmethyl, m-alkoxy groups (m with respect to the C—P bond), $SO_3H$ groups and cyano groups. Very good results have been obtained with chlorine atoms.

Examples of suitable phosphines are tri-m-methoxyphenyl-phosphine, tri(o-fluorophenyl-)phosphine, tri(m-trichloromethylphenyl)phosphine and in particular tri(p-chlorophenyl)phosphine. The phosphine is used in a quantity of at least 5 mol, preferably 10—150 mol per gram atom of palladium. If the palladium compound already contains phosphine, this should be taken into account when calculating the amount of phosphine to be used.

In the process according to the invention the carbon monoxide may be used pure or diluted with an inert gas, such as nitrogen, noble gases or carbon dioxide. Generally the presence of more than 10%v of hydrogen is undesirable, since under the reaction conditions it may cause hydrogenation of the ethylenically unsaturated compound. Generally preference is given to the use of carbon monoxide or a carbon monoxide-containing gas which contains less than 5%v of hydrogen.

The process according to the invention is preferably carried out at a temperature in the range of from 50 to 200°C, in particular from 75 to 150°C. The overall pressure preferably lies in the range of from 1 to 100, in particular 20 to 75, bar.

The molar ratio of the ethylenically unsaturated

compound to carboxylic acid is not critical. The molar ratio between carboxy groups and ethylenically unsaturated bonds may lie for instance between 0.1:1 and 10:1. When using a monoolefin and a monobasic acid, preference is usually given to the use of an excess of the monobasic acid mentioned. However, when using a polybasic acid to prepare a polyanhydride, it will generally be necessary to use an excess of the ethylenically unsaturated compound.

The process according to the invention may be carried out batchwise, continuously or semi-continuously. Generally there is no need for the use of a solvent since usually there will be an excess of one of the reactants which may serve as a solvent as well. If required, however, a solvent may be used, for instance sulphoxides, for example dimethyl sulphoxide and diethyl sulphoxide; sulphones, for example diisopropyl sulphone and tetrahydrothiophene-1,1-dioxide (also referred to as "sulfolane"), chloroform and ethers, for example diglyme (dimethyl ether of diethylene glycol), diphenyl ether or diisopropyl ether.

The following Examples further illustrate the invention. The experiments were carried out in a 250 ml magnetically stirred Hastelloy C autoclave ("Hastelloy" is a trade name).

Example 1

The autoclave was charged with toluene (30 ml), acetaldehyde (20 ml), propionic acid (10 ml), palladium (II) acetate (0.2 mmol), tri(p-chlorophenyl)phosphine (10 mmol) and p-toluene-sulphonic acid (3 mmol). The autoclave was flushed with carbon monoxide, filled with ethene at a pressure of 30 bar and carbon monoxide at a pressure of 30 bar, sealed and heated to a temperature of 110°C. After a reaction time of 5 h the contents of the autoclave were analysed by gas-liquid chromatography. The conversion of acetaldehyde was 60%, which is the maximal possible conversion, with a selectivity to ethylidene dipropionate of 90%, the balance being propionic anhydride (5%) and vinyl propionate (5%).

Example 2

Example 1 was repeated with the difference that tri(p-chlorophenyl)phosphine (10 mmol) was replaced with triphenylphosphine (10 mmol). The conversion of acetaldehyde was 25%, with a selectivity to ethylidene dipropionate of 30% and to propionic anhydride of 60%.

Example 3

Example 1 was repeated with the difference that 20 ml instead of 10 ml of propionic acid was used. The conversion of acetaldehyde was 65% (maximal possible conversion 80%) with a selectivity to ethylidene dipropionate of 72%, the balance being propionic anhydride (25%) and vinyl propionate (3%).

Example 4

Example 1 was repeated with the difference that tri(p-chlorophenyl)phosphine (10 mmol) was replaced with triphenylphosphine (50 mmol) and that 15 mmol instead of 3 mmol of p-toluenesulphonic acid were used. After a reaction time of 2 h at 125°C acetaldehyde conversion was 56%, with a selectivity to ethylidene dipropionate of 48% and to propionic anhydride of 50%.

Example 5

Example 1 was repeated with the difference that 50 mmol instead of 10 mmol of tri(p-chlorophenyl)phosphine and that 15 mmol instead of 3 mmol of p-toluenesulphonic acid was used. After a reaction time of 5 h at 125°C acetaldehyde conversion was 60%, with a selectivity to ethylidene dipropionate of 89%, to propionic anhydride of 8% and to vinyl propionate of 3%.

**Claims**

1. A process for the preparation of an alkylidene diester, which process comprises reacting an ethylenically unsaturated compound with carbon monoxide, a carboxylic acid and an aldehyde in the presence of a catalytic system prepared by combining palladium or a palladium compound, at least 5 mol of a phosphine $PR^1R^2R^3$ in which $R^1$, $R^2$ and $R^3$ each represent an optionally substituted aryl group, per gram atom of palladium, and an acid with a $pK_a<2$ (at 18°C in aqueous solution) as a promoter, except hydrohalogenic and carboxylic acids.

2. A process as claimed in claim 1, characterized in that an acid with a non-co-ordinating anion is used as promoter.

3. A process as claimed in claim 1 or 2, characterized in that a sulphonic acid or an acid that can be formed by interaction of a Lewis acid with a Broensted acid is used as promoter.

4. A process as claimed in claim 1 or 2, characterized in that an acid having the general formula

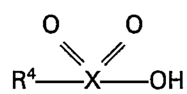 I

wherein X represents sulphur or chlorine and, if X is chlorine, $R^4$ represents oxygen and if X is sulphur, $R^4$ represents an OH group or an optionally substituted hydrocarbon group, is used as promoter.

5. A process as claimed in claim 4, characterized in that the optionally substituted hydrocarbon group represented by $R^4$ is an alkyl, aryl, aralkyl or alkaryl group having 1—30 carbon atoms.

6. A process as claimed in claim 4 or 5, characterized in that the acid is p-toluenesulphonic acid or trifluoromethanesulphonic acid.

7. A process as claimed in any one of the preceding claims, characterized in that the acid with a $pK_a<2$ is present in a quantity of 0.01—150, in particular 1—50, equivalents per gram atom of palladium.

8. A process as claimed in any one of the preceding claims, characterized in that the ethylenically unsaturated compound is an unsubstituted or substituted alkene or cycloalkene having 3—20 carbon atoms and 1—3 double bonds per molecule.

9. A process as claimed in any one of the preceding claims, characterized in that the carboxylic acid has not more than than 20 carbon atoms per molecule.

10. A process as claimed in any one of the preceding claims, characterized in that the aldehyde is an unsubstituted or substituted alkanal having 2—30 carbon atoms per molecule.

11. A process as claimed in any one of the preceding claims, characterized in that the aryl groups represented by the groups $R^1$, $R^2$ and $R^3$ have 6—14 carbon atoms.

12. A process as claimed in any one of the preceding claims, characterized in that each of the aryl groups carry an electron-withdrawing substituent.

13. A process as claimed in claim 12, characterized in that tri(p-chlorophenyl)phosphine is used in the catalytic system.

14. A process as claimed in any one of the preceding claims, characterized in that 10—150 mol phosphine is used per gram atom palladium.

15. A process as claimed in any one of the preceding claims, characterized in that the process is carried out at a temperature in the range of from 50 to 200°C.

16. A process as claimed in any one of the preceding claims, characterized in that the process is carried out at a pressure in the range of from 20 to 75 bar.

**Patentansprüche**

1. Verfahren zur Herstellung eines Alkylidendiesters, wobei das Verfahren umfaßt die Umsetzung einer ethylenisch ungesättigten Verbindung mit Kohlenmonoxid, einer Carbonsäure und einem Aldehyd in Gegenwart eines katalytischen Systems, das hergetellt worden ist durch Kombination von Palladium oder einer Palladiumverbindung, mindestens 5 mol eines Phosphins $PR^1R^2R^3$, bei dem $R^1$, $R^2$ und $R^3$ jeweils eine gegebenenfalls substituierte Arylgruppe bedeuten, pro gAtom Palladium, und einer Säure mit einem $pK_a$-Wert<2 (bei 18°C in wäßriger Lösung) als Promotor, mit Ausnahme von Halogenwasserstoff- und Carbonsäuren.

2. Verfahren nach Anspruch 1, dadurch gekennzeichnet, daß eine Säure mit einem nicht-koordinierenden Anion als Promotor angewandt wird.

3. Verfahren nach Anspruch 1 oder 2, dadurch gekennzeichnet, daß eine Sulfonsäure oder eine Säure, die durch Wechselwirkung zwischen einer Lewis-Säure und einer Broensted-Säure gebildet werden kann, als Promotor angewandt wird.

4. Verfahren nach Anspruch 1 oder 2, dadurch gekennzeichnet, daß eine Säure der allgemeinen Formel

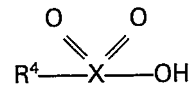

I

in der X Schwefel oder Chlor bedeutet und, wenn X Chlor ist, $R^4$ Sauerstoff und, wenn X Schwefel ist, $R^4$ eine OH-Gruppe oder eine gegebenenfalls substituierte Kohlenwasserstoffgruppe bedeutet, als Promotor angewandt wird.

5. Verfahren nach Anspruch 4, dadurch gekennzeichnet, daß die durch $R^4$ angegebene, gegebenenfalls substituierte Kohlenwasserstoffgruppe eine Alkyl-, Aryl-, Aralkyl- oder Alkarylgruppe mit 1—30 Kohlenstoffatomen ist.

6. Verfahren nach Anspruch 4 oder 5, dadurch gekennzeichnet, daß die Säure p-Toluolsulfonsäure oder Trifluormethansulfonsäure ist.

7. Verfahren nach einem der vorangehenden Ansprüche, dadurch gekennzeichnet, daß die Säure mit einem $pK_a$-Wert<2 in einer Menge von 0,01—150, insbesondere 1—50, Äquivalent pro gAtom Palladium vorhanden ist.

8. Verfahren nach einem der vorangehenden Ansprüche, dadurch gekennzeichnet, daß die ethylenisch ungesättigte Verbindung ein unsubstituiertes oder substituiertes Alken oder Cycloalken mit 2—30 Kohlenstoffatomen und 1—3 Doppelbindungen im Molekül ist.

9. Verfahren nach einem der vorangehenden Ansprüche, dadurch gekennzeichnet, daß die Carbonsäure nicht mehr als 20 Kohlenstoffatome im Molekül enthält.

10. Verfahren nach einem der vorangehenden Ansprüche, dadurch gekennzeichnet, daß der Aldehyd ein unsubstituiertes oder substituiertes Alkanal mit 2—30 Kohlenstoffatomen im Molekül ist.

11. Verfahren nach einem der vorangehenden Ansprüche, dadurch gekennzeichnet, daß die durch die Gruppen $R^1$, $R^2$ und $R^3$ angegebenen Arylgruppen 6—14 Kohlenstoffatome besitzen.

12. Verfahren nach einem der vorangehenden Ansprüche, dadurch gekennzeichnet, daß jede der Arylgruppen einen Elektronen-abziehenden Substituenten enthält.

13. Verfahren nach Anspruch 12, dadurch gekennzeichnet, daß Tri(p-chlorphenyl)phosphin in dem katalytischen System angewandt wird.

14. Verfahren nach einem der vorangehenden Ansprüche, dadurch gekennzeichnet, daß 10—150 mol Phosphin pro gAtom Palladium angewandt werden.

15. Verfahren nach einem der vorangehenden Ansprüche, dadurch gekennzeichnet, daß das Verfahren bei einer Temperatur im Bereich von 50 bis 200°C durchgeführt wird.

16. Verfahren nach einem der vorangehenden Ansprüche, dadurch gekennzeichnet, daß das Verfahren bei einem Druck im Bereich von 20 bis 75 bar durchgeführt wird.

**Revendications**

1. Un procédé pour la préparation d'un diester d'alkylidène, procédé qui comprend la réaction

d'un composé éthyléniquement insaturé avec l'oxyde de carbone, un acide carboxylique et un aldéhyde en présence d'un système catalytique préparé en combinant du palladium ou un composé du palladium, au moins 5 moles d'une phosphine $PR^1R^2R^3$, où $R^1$, $R^2$ et $R^3$ représentent chacun un groupe aryle éventuellement substitué, par atome-gramme de palladium, et un acide ayant un $pK_a$ inférieur à 2 (à 18°C en solution aqueuse) comme promoteur, à l'exception des acides halogénhydriques et carboxyliques.

2. Un procédé selon la revendication 1, caractérisé en ce qu'un acide ayant un anion non-coordinateur est utilisé comme promoteur.

3. Un procédé selon la revendication 1 ou 2, caractérisé en ce qu'un acide sulfonique ou un acide qui a été formé par interaction d'un acide de Lewis avec un acide de Broensted est utilisé comme promoteur.

4. Un procédé selon la revendication 1 ou 2, caractérisé en ce qu'un acide ayant la formule générale

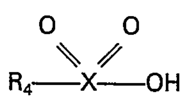

I

où X représente du soufre ou du chlore et, si X est du chlore, alors $R^4$ de l'oxygène et, si X est du soufre, alors $R^4$ représente un groupe OH ou un groupe d'hydrocarbure éventuellement substitué, est utilisé comme promoteur.

5. Un procédé selon la revendication 4, caractérisé en ce que le groupe d'hydrocarbure éventuellement substitué représenté par $R^4$ est un groupe alkyle, aryle, aralkyle ou alkaryle ayant 1—30 atomes de carbone.

6. Un procédé selon la revendication 4 ou 5, caractérisé en ce que l'acide est de l'acide p-toluènesulfonique ou de l'acide trifluorométhane-sulfonique.

7. Un procédé selon l'une quelconque des revendications précédentes, caractérisé en ce que l'acide ayant un $pK_a$ inférieur à 2 est présent à raison de 0,01—150, en particulier 1—50 équivalents par atome-gramme de palladium.

8. Un procédé selon l'une quelconque des revendications précédentes, caractérisé en ce que le composé éthyléniquement insaturé est un alkène ou cycloalkène substitué ou non ayant 2—30 atomes de carbone et 1—3 doubles liaisons par molécule.

9. Un procédé selon l'une quelconque des revendications précédentes, caractérisé en ce que l'acide carboxylique n'a pas plus de 20 atomes de carbone par molécule.

10. Un procédé selon l,une quelconque des revendications précédentes, caractérisé en ce que l'aldéhyde est un alkanal substitué ou non ayant 2—30 atomes de carbone par molécule.

11. Un procédé selon l'une quelconque des revendications précédentes, caractérisé en ce que les groupes aryle représentés par les groupes $R^1$, $R^2$ et $R^3$ ont 6—14 atomes de carbone.

12. Un procédé selon l'une quelconque des revendications précédentes, caractérisé en ce que chacun des groupes aryle porte un substituant qui attire les électrons.

13. Un procédé selon la revendication 12, caractérisé en ce qu'on utilise la tri(p-chlorophényl)phosphine dans le système catalytique.

14. Un procédé selon l'une quelconque des revendications précédentes, caractérisé en ce qu'on utilise 10—150 moles de phosphine par atome-gramme de palladium.

15. Un procédé selon l'une quelconque des revendications précédentes, caractérisé en ce que le procédé est mis en oeuvre à une température comprise entre 50 et 200°C.

16. Un procédé selon l'une quelconque des revendications précédentes, caractérisé en ce que le procédé est mis en oeuvre à une pression comprise entre 20 et 75 bars.